(19) Europäisches Patentamt
European Patent Office
Office européen des brevets

(11) EP 0 859 653 B1

(12) # EUROPÄISCHE PATENTSCHRIFT

(45) Veröffentlichungstag und Bekanntmachung des Hinweises auf die Patenterteilung:
**04.08.1999 Patentblatt 1999/31**

(21) Anmeldenummer: **96945840.5**

(22) Anmeldetag: **26.10.1996**

(51) Int. Cl.$^6$: **B01D 3/00**, B01J 8/02, B01J 8/04

(86) Internationale Anmeldenummer:
**PCT/DE96/02056**

(87) Internationale Veröffentlichungsnummer:
**WO 97/16243 (09.05.1997 Gazette 1997/20)**

(54) **VERFAHREN ZUR CHEMISCHEN UMSETZUNG VON STOFFEN IN EINER REAKTIONSKOLONNE**

METHOD OF CHEMICALLY REACTING SUBSTANCES IN A REACTION COLUMN

PROCEDE POUR FAIRE REAGIR CHIMIQUEMENT DES SUBSTANCES DANS UNE COLONNE DE REACTION

(84) Benannte Vertragsstaaten:
**BE DE ES FR GB IT NL**

(30) Priorität: **04.11.1995 DE 19541213**

(43) Veröffentlichungstag der Anmeldung:
**26.08.1998 Patentblatt 1998/35**

(73) Patentinhaber:
**RWE-DEA AKTIENGESELLSCHAFT FÜR MINERALOEL UND CHEMIE**
**22297 Hamburg (DE)**

(72) Erfinder:
- **HOFFMANN, Ulrich**
**D-37154 Northeim (DE)**
- **KUNZ, Ulrich**
**D-38678 Clausthal-Zellerfeld (DE)**

(74) Vertreter: **Schupfner, Gerhard D.**
**Müller, Schupfner & Gauger,**
**Karlstrasse 5**
**21244 Buchholz (DE)**

(56) Entgegenhaltungen:
**EP-A- 0 670 178**
**WO-A-94/19079**
**DE-A- 4 234 779**
**DE-C- 976 413**
**US-A- 2 384 793**
**US-A- 3 124 526**
**US-A- 5 449 501**

EP 0 859 653 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung bezieht sich auf ein Verfahren in einer Reaktionskolonne zur chemischen Umsetzung von Stoffen, deren Reaktion durch eine ungünstige Gleichgewichtslage der Bildungsreaktion oder ein vorgelagertes Gleichgewicht beschränkt ist, wobei während der Umsetzung durch einen oder mehrere Hilfsstoffe kontinuierlich ein oder mehrere abzutrennende Stoffe aus der Reaktionsmischung entfernt werden.

Für Prozesse, bei denen aufgrund der Reaktion zugrundeliegenden chemischen Gleichgewichtslage der erreichbare Umsatz beschränkt ist, hat sich in der Vergangenheit die Reaktivdestillation bewährt. Neben der Anwendung bei der Herstellung von Ethern, die z.B. als Antiklopfmittel in Kraftstoffen Verwendung finden, ist ein Anwendungsgebiet die Veresterung. Stand der Technik ist es, eine Veresterung als Reaktivdestillation durchzuführen, um das Reaktionswasser durch Destillation kontinuierlich abzutrennen, siehe z.B. J. Krafczyk und J. Gmehling in Chem.-Ing.-Tech. 1372, 66, 1994 und C. Breucker, V. Jordan, M. Nitsche und B. Gutsche in Chem.-Ing.-Tech. 430, *67*, 1995. Bei einer konventionellen Reaktivdestillation sind die Temperaturen in der Kolonne so gewählt, daß bis auf das Sumpfprodukt alle Stoffe bis zum Sieden erhitzt werden.

Bei diesen bekannten Verfahren wird die Abtrennung des Reaktionswassers aber immer durch einen Reaktanden bewirkt, der meist im großen Überschuß zugeführt werden muß. Notwendigerweise ist daher die zur Trennung erforderliche Temperatur immer gleich oder größer als die Siedetemperatur der Reaktanden unter den gegebenen Druckverhältnissen. Die Reaktanden sind in diesem Fall der Hilfsstoff und ein weiterer Stoff, wobei im Falle der Veresterung der Alkohol, wem er im Überschuß verwendet wird, gleichzeitig als Reaktand für die Veresterungsreaktion und als Hilfsstoff zur Entfernung der Reaktionswassers dient. Darüber hinaus muß zur effektiven Trennung das Einsatzverhältnis der Reaktanden festgelegt sein. C. Breucker et. al. weisen darauf hin, daß die Abtrennung des Reaktionswassers bei geringen Reaktandenüberschüssen eine verfahrenstechnische Herausforderung ist. Bisher hat man versucht dieses Problem durch eine "Batch"-Verfahrensführung oder eine Verfahrensführung in einer Kaskade mit mehreren Reaktoren zu lösen.

Eine weithin bekannte Ausführungsform des Standes der Technik ist die einstufig geführte Veresterung mit Hilfe eines Schleppmittels unter Ausnutzung der Azeotropbildung, siehe z.B. Organikum, VEB Deutscher Verlag der Wissenschaften, Berlin 1977, S. 71-73. Auch Verfahren zur Herstellung von Estern aus Carbonsäuren und Alkoholen mit kontinuierlicher Entfernung des Kondensationswassers mit Schleppmitteln wie Benzol, Methanol und Butylalkohol in einer Kolonne sind aus DE 976 413-B bekannt. In US 2,384,793 wird als Schleppmittel Butan eingesetzt.

In der WO 94/19079 wird ein Verfahren zur Herstellung von Bisphenol-A aus Aceton und Phenol unter Wasserabspaltung offenbart. Das entstehende Reaktionswasser wird durch ein inertes Strippgas ausgetragen. In dem Verfahren der WO 94/19079 wird ein Reaktor eingesetzt, der einen flüssigen Katalysator oder einen partikulären Katalysator in Suspension und im Reaktor selbst Böden, Ablaufschächte und/oder Rückhaltesiebe enthält.

Der Erfindung liegt die Aufgabe zugrunde, ein Verfahren bereitzustellen, um für Gleichgewichtsraktionen den Umsatz günstig zu beeinflussen und insbesondere einen gebildeten abzutrennender Stoff durch einen Hilfsstoff in einer Kolonne kontinuierlich, schonend und besonders effektiv zu entfernen.

Überraschend wurde gefunden, daß in Kolonnen mit Festbettkatalysatoren zu diesem Zweck Permanentgase als Strippgase besonders geeignet sind. Der fördernde Effekt des Stripprozesses soll den Reaktionsablauf begünstigen, und gleichzeitig den hohen stöchiometrischen Überschuß eines Reaktanden vermeiden. Der Hilfsstoff soll nach vollbrachter Aufgabe einfach zurückgeführt werden können. Durch den Einsatz dieses Verfahrens sollen die in den Verfahren nach dem Stand der Technik notwendigerweise vorhandenen Kopplungen der Betriebsparameter wie Betriebstemperatur-Druck-Siedetemperatur-Einsatzverhältnis vermieden werden.

Erfindungsgemäß wird diese Aufgabe gelöst durch ein Verfahren in einer Reaktionskolonne zur chemischen Umsetzung von Stoffen, deren Reaktion durch eine ungünstige Gleichgewichtslage der Bildungsreaktion oder ein vorgelagertes Gleichgewicht beschränkt ist, wobei während der Umsetzung durch einen oder mehrere Hilfsstoffe kontinuierlich ein oder mehrere abzutrennende Stoffe aus der Reaktionsmischung entfernt werden, indem man durch die Reaktionskolonne, in der ein Feststoff-Katalysator in Form eines Festbettes vorliegt, ein Strippgas, nämlich ein Permanentgas oder ein Gemisch von Permanentgasen als Hilfsstoff, leitet und Temperatur und Druck so einstellt, daß alle Edukte als Flüssigkeiten zu oder als Lösungen von Feststoffen und der oder die abzutrennenden Stoffe überwiegend gasförmig/dampfförmig in der Reaktionskolonne vorliegen. Das Strippgas wird vorzugsweise im Gegenstrom zum Flüssigkeitsstrom geführt. Strippvorgang und Umsetzung finden simultan in der Reaktionskolonne statt. Der Katalysator kann in der Form einer geordneten Packung oder ungeordneten Schüttung eingebracht werden.

Der kontinuierlich zugeführte Hilfsstoff ist ein Permanentgas. Permanentgase im Sinne der Erfindung sind Gase, die sich bei Temperaturen von über -30 °C nicht mehr verflüssigen lassen. Permanentgase im Sinne der Erfindung sind Gase, die aus 5 oder weniger atomigen Molekülen bestehen sowie deren Mischungen. Dies können unter anderem auch C1-Kohlenwasserstoffe sein. Besonders geeignet sind aber Stoffe, deren Moleküle aus lediglich einem Element bestehen, wie Stickstoff ($N_2$) oder Wasserstoff ($H_2$), besonders bevorzugt ist Wasserstoff Diese Permanentgase dienen als Strippgas für das abzutrennende Produkt. Der Unterschied zu einem konventionellen Schleppmittel

besteht darin, daß das Strippgas bereits gasförmig vorliegt, während das Schleppmittel zunächst selbst in den Gaszustand überführt werden muß. Dies erfordert einen entsprechenden Aufwand an Verdampfungswärme. Weiterhin ist man darauf angewiesen, daß das dem Gleichgewicht zu entziehende Produkt ebenfalls in der Gasphase vorliegt. Dieses kann im vorliegenden Fall auch teilweise flüssig vorliegen.

Von Vorteil ist das erfindungsgemäße Verfahren immer dann, wenn nicht alle reagierenden Stoffe oder Produkte bis zum Siedepunkt erhitzt werden können, weil sie sich dann zersetzen oder störende Nebenreaktionen auftreten. In solchen Fällen kann die aus dem Stand der Technik bekannte Reaktivdestillation nicht angewendet werden, weil sich die Reaktanden zersetzen würden. Deshalb eignet sich dieses neuartige Verfahren besonders für alle Umsetzungen von natürlichen oder temperaturempfindlichen Stoffen, bei denen die ständige Entfernung eines niedermolekularen Reaktionsproduktes günstig auf den Reaktionsablauf wirkt, die Reaktanden oder Wertprodukte selbst aber nicht bis zum Siedepunkt erhitzt werden dürfen.

Der Katalysator ist ein Festbett-Katalysator. In einer bevorzugten Variante wird die Reaktivkolonne im Rieselregime betrieben. Dann werden etwa 30-60 Vol% bevorzugt 50 Vol.% der Reaktionskolonne als freier Gasraum von dem Strippgas genutzt, während 30 bis 50 Vol.% bevorzugt 40 Vol.% der Kolonne von dem Feststoff, nämlich dem Festbett-Katalysator, eingenommen werden. Die herunterrieselnde Flüssigkeit nimmt den Rest, bevorzugt 10 Vol.% oder weniger, des Reaktionsraumes ein. Hohe Katalysatorkonzentrationen können unabhängig von sonstigen Betriebsparametern über weite Bereiche eingestellt werden. Gegenüber dem Stand der Technik ist das Verhältnis Katalysator zu Flüssigphase überraschend hoch.

Bei Verwendung eines Festbettes kann die Verweilzeit der Flüssigphase durch die Gasgeschwindigkeit des Strippgases eingestellt werden. Bei höheren Volumengeschwindigkeiten des Strippgases ist die Verweilzeit der Flüssigphase hoch. Der Strippgasdurchsatz ist ohne nachteilige Beeinflussung der Prozeßführung über große Bereiche einstellbar.

Die Wirkung des Strippgases kann durch einen weiteren Hilfsstoff modifiziert werden. Dieser weitere Hilfsstoff ist dabei ein konventioneller Schlepper wie Benzol, Toluol oder Hexan, besonders bevorzugt Hexan. In diesem Fall entfernt das Strippgas den abzutrennenden Stoff zusammen mit dem Schlepper, der unter den Bedingungen der Strippkolonne gasförmig vorliegt.

Werden neben Permanentgasen zusätzlich Schlepper als Hilfsstoffe eingesetzt, so übersteigt der Anteil des Permanentgase in der Reaktionskolonne in jedem Falle 80 mol% der eingesetzten Hilfsstoffe, während der Anteil des Schleppers dementsprechend den sich zu 100 mol% ergänzenden Anteil bildet. Der Mol-Anteil des Schleppers ist um mindestens Faktor 4 kleiner als der Anteil der Permanentgase in der Reaktionskolonne.

Die Reaktionen, für die dieses neuartige Verfahren angewendet werden kann, laufen allgemein nach folgender Gleichung ab:

$$A+B+C+... \leftrightarrows U+V+W+...$$

Dabei ist mindestens eines der Produkte - der abzutrennende Stoff - unter den Reaktionsbedingungen des Reaktionsstrippers verdampfbar, bzw. läßt sich durch das Strippgas in die Dampfphase überführen. Die Reaktion ist eine Gleichgewichtsreaktion oder wird durch ein vorgelagertes Gleichgewicht in ihrem Ablauf beeinflußt. Beispiele sind folgende Reaktionstypen:

**Aldolkondensation**

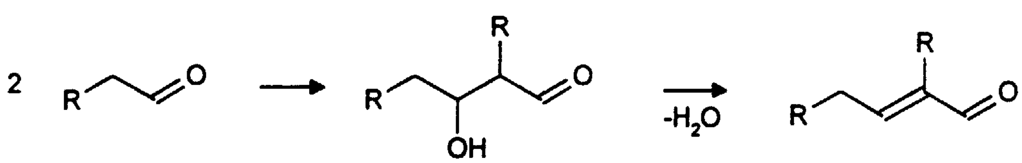


**Knoevenagelkondensation**

Die Knoevenagelkondensation ist ein Spezialfall der Aldolreaktion mit anschließender Aldolkondensation. Hierbei werden Methylenkomponenten mit besonders großer CH-Acidität wie z. B. Malonsäure, Malonhalbester, Malonester, Cyanessigsäure, Cyanessigsäureester und andere mit Aldehyden und Ketonen umgesetzt.

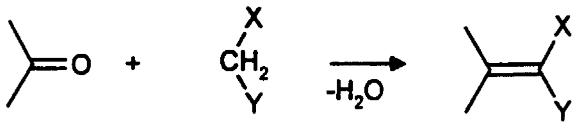


(X,Y=COR,COOR,COOH,CN, $NO_2$)

Stoffbeispiel:

Die Reaktanden sind temperaturempfindlich und sieden höher als der Hilfsstoff Das abzutrennende Wasser (abzutrennender Stoff) wird durch das Strippgas entfernt oder das abzutrennende Wasser bildet mit einem zusätzlich zu verwendenden Schlepper wie Hexan, Benzol oder Toluol, bevorzugt aber Hexan, ein Azeotrop, wobei in diesem Fall das Permanentgas zusammen mit Schlepper und Wasser den Reaktionsstripper über Kopf verläßt. Das Produkt Benzalmalonsäurediethylester ist der am höchsten siedende Stoff (186°C bei 18 Torr). Dieser verläßt die Kolonne am Sumpf.

| Reaktanden | Sdp. | Katalysator: | Hilfsstoff |
|---|---|---|---|
| Benzaldehyd<br>Malonsäurediethylester | 177°C<br>94-96°C /11 Torr | Basen, z.B. basische Ionenaustauscher, Karbonate der Alkali- und Erdalkalimetalle | Strippgas oder Strippgas + Hexan |

**Darstellung von Enaminen**

Bei der Umsetzung von Aldehyden und Ketonen mit sekundären Aminen entstehen Enamine. Dies ist eine wichtige Route, um Zwischenprodukte für die organische Synthese zu gewinnen. Ein bedeutendes Anwendungsgebiet ist die Erzeugung von Heterozyklen (bei Einsatz entsprechender Reaktanden).

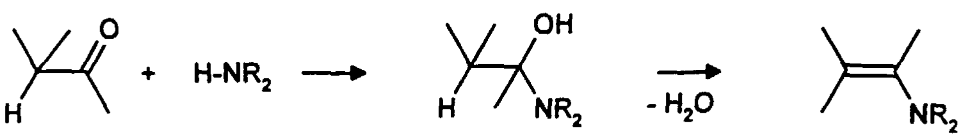


Stoffbeispiel:

Wie im vorstehenden Beispiel ist das Produkt ß-Benzylaminocrotonsäureethylester (Siedepunkt 140°C bei 0,5 Torr) der am höchsten siedende Stoff. Strippgas oder Strippgas zusammen mit Schlepper als Hilfsstoff transportieren das Wasser (abzutrennender Stoff) zum Kopf des Apparates. Das Produkt verläßt den Reaktionsstripper am Sumpf.

| Reaktanden | Sdp. | Katalysator: | Hilfsstoff |
|---|---|---|---|
| Acetessigsäureethylester<br>Benzylamin | 68-79°C bei 11 Torr<br>70-71°C bei 10 Torr | Säuren, z. B. saure Ionentauscher oder Toluolsulfonsäure | Strippgas oder Strippgas + Hexan |

**Reaktionen von Carbonylverbindungen mit Basen**

Reaktionen von Carbonsäuren mit Aminen zu Carbonsäureamiden:

Die Bildung des Säureamids wird durch das kontinuierliche Entfernen des Wassers (abzutrennender Stoff) im Reaktionsstripper begünstigt. Der mit Wasser angereicherte Hilfsstoff verläßt den Apparat am Kopf, das Produkt wird am Sumpf abgeleitet.

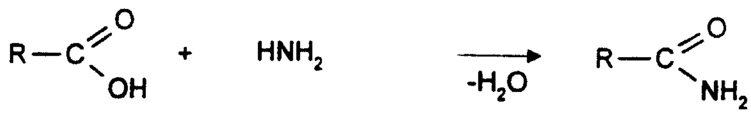


Stoffbeispiel:

| Reaktanden | Sdp. | Katalysator: | Hilfsstoff |
|---|---|---|---|
| Capronsäure<br>Dibutylamin | 205°C<br>159-161°C | Säuren, z. B. saurer Ionentauscher | Strippgas oder Strippgas + Hexan |

**Guerbet-Reaktion**

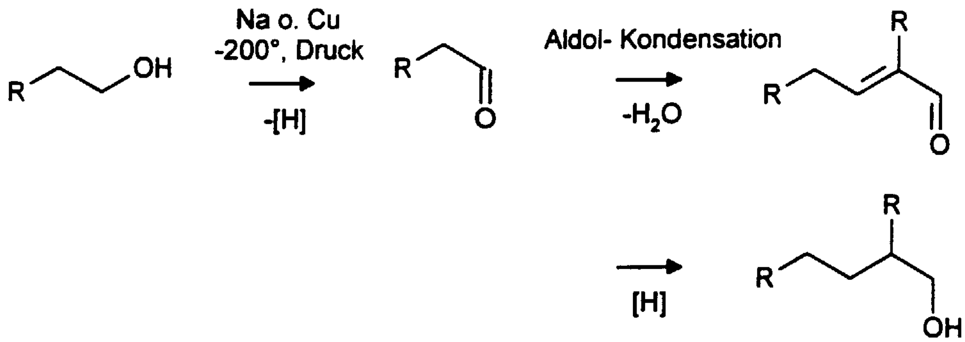

**Veresterung von Carbonsäuren**

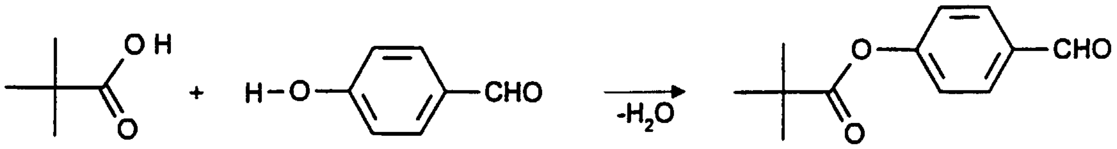

Stoffbeispiel:

| Reaktanden | Katalysator: | Hilfsstoff |
|---|---|---|
| Isobuttersäure oder Pivalinsäure mit Hydroxybenzaldehyd | Säuren, z. B. saurer Ionentauscher | Wasserstoff als Strippgas ggf. zusammen mit Benzol, Toluol oder bevorzugt Hexan |

**Reaktion von Fettsäuren mit Fettalkoholen zu Fettsäureestern**

Ein Vorteil des erfindungsgemäßen Verfahrens ist es , daß bei Verwendung von Permanentgasen als Hilfsstoff oft einfachere Kühler in den Kondensatoren eingesetzt werden können, da die die Stofftrennung bewirkenden physikalischen Daten der Permanentgase in vielen Fällen weit entfernt sind von den Stoffdaten der abzutrennenden Stoffe (Siedepunkt, Kondensationswärmen, Verdampfungswärmen). Dies spart auch Energiekosten für die Vorheizung, denn es braucht nur die Wärme zur Temperaturerhöhung des Gases auf Reaktionstemperatur eingebracht werden, nicht aber die Verdampfungswärme eines flüssigen Schleppers.

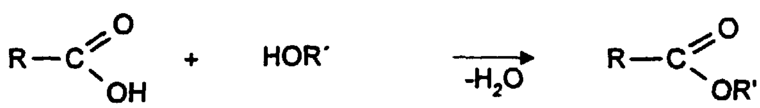

Stoffbeispiel:

| Reaktanden | Katalysator: | Hilfsstoff |
|---|---|---|
| Fettsäure C6-C22, bevorzugt C8-C22<br>Fettalkohole C1-C22, bevorzugt C8-C22 | Säuren, z. B. saurer Ionentauscher | Wasserstoff oder Stickstoff als Strippgas ggf. zusammen mit Hexan (Azeotropbildner) |

Vorteilhafte Ausgestaltung des Verfahrens (allgemein, für alle Reaktionsvarianten): Eine besonders vorteilhafte Variante des Verfahrens ist in Abbildung 1 gezeigt. Hierbei wird ein Gemisch der Reaktanden im oberen Teil des Reaktionsstrippers zugeführt. Während des durch die Schwerkraft bewirkten Flusses der flüssigen Reaktionsmischung nach unten über die Stripperfüllung erfolgt die chemische Reaktion. Die Füllung kann aus einem festen bzw. geträgerten Katalysator wie auch aus mindestens einer teilweise inerten Packung bestehen. Das gebildete abzutrennende Produkt verdampft und wird durch einen vorzugsweise von unten im Gegenstrom zugeführten Hilfsstoffstrom aus der Reaktionszone entfernt. Der mit dem abzutrennenden Stoff angereicherte Hilfsstoff verläßt den Reaktionsstripper über Kopf. Nach Auskondensation des abzutrennenden Stoffes kann der Hilfsstoff wieder dem Apparat oder einer nachgeschalteten Verfahrensstufe als Reaktant zugeführt werden. Das gewünschte flüssige Reaktionsprodukt verläßt den Reaktionsstripper am Sumpf.

Durch Entfernung des Reaktionswassers mit einem Hilfsstoff gelingt es, die Reaktion zugunsten des gewünschten Produktes z.B. Fettsäureester ablaufen zu lassen. Nach Abbildung 1 gelangt eine Mischung von z.B. Fettsäure mit Fettalkohol im oberen Teil in den Reaktionsstripper. Alternativ können aber auch die Edukte in einer Vorreaktion teilumgesetzt werden, mit dem Vorteil, für den selben Umsatz in der Kolonne jetzt eine kürzere Kolonnenlänge des Reaktionsstrippers zu benötigen. Das Produkt der Vorreaktion wird in diesem Fall in den Reaktionsstripper eingeschleust.

Die Temperatur und der Druck sind im Reaktionsstripper so eingestellt, daß beide Reaktanden wie auch das Produkt (z.B. Fettsäureester) flüssig sind, die abzutrennende Komponente z.B. Wasser aber bereits verdampft und überwiegend dampfförmig vorliegt.

Von unten strömt als Strippgas z.B. vorgeheizter, trockener Wasserstoff in die Anlage. Dieser strömt nach oben zum Kopf des Reaktionsstrippers. Auf seinem Weg dorthin nimmt er die abzutrennende Komponente z.B. Wasser mit. Der mit der abzutrennende Komponente z.B. Wasser angereicherte Wasserstoff verläßt den Apparat am Kopf. Von dort gelangt er in einen Kondensator, in dem er von der abzutrennende Komponente z.B. Wasser getrennt wird. Wahlweise kann der Wasserstoff wieder in den Reaktionsstripper geleitet werden oder einer anderen Verwendung zugeführt werden. Die Einspeisung eines der Reaktanden, bevorzugt der Alkohol, kann in den Reaktionsstripper auch durch eine Seiteneinspeisung erfolgen. Dadurch ergibt sich eine zusätzliche Möglichkeit, mit der die genannte Reaktion in Richtung der Produkte verschoben werden kann. Zusätzlich erlaubt diese Verfahrensvariante durch Vorheizen der Seitenströme eine gezielte Beeinflussung des Temperaturprofiles in dem Reaktionsstripper. Ein besonderes Kennzeichen des erfindungsgemäßen Verfahrens ist es, daß die Edukte, z.B. die Carbonsäure, bzw. im Falle der Umesterung der Carbonsäureester, und der Alkohol, vorteilhafterweise äquimolar eingesetzt werden können. Wenn jedoch eine überstöchiometrische Umsetzung erfolgen soll, wird der Alkohol im Überschuß zugesetzt. Das Produkt, z.B. der Fettsäureester, wird im unteren Teil der Kolonne abgeleitet.

Der Reaktionsstripper ist mit einem Feststoff-Katalysator ausgestattet. Dadurch kann die Reaktionsgeschwindigkeit der allein schon durch das Strippen beschleunigten Reaktion weiter gesteigert werden. Dieser Katalysator ist zweckmäßigerweise bei Verwendung saurer Katalysatoren wie z. B. im Falle der Veresterung eine feste Säure, z.B. ein saurer Ionentauscher. Zum Einsatz eignen sich besonders solche Ionentauscher, die einen Einsatz auch bei hohen Reaktionstemperaturen von bis zu 230°C erlauben. Im Falle wasserempfindlicher Katalysatoren - wenn z.B. das Reaktionswasser vom Katalysator adsorbiert wird - kann nach mehreren Zyklen zur Umsatzerhöhung eine Reaktivierung des Katalysators durch Trocknung erfolgen.

Die Betriebsbedingungen liegen für die Betriebstemperaturen im Bereich von 20 bis 300 °C, vorzugsweise 100-230 °C, und der Betriebsdruck im Bereich von Unterdruck bis 50 bar, vorzugsweise 1,5 - 15 bar. Im Falle der Veresterung hat die Querschnittsbelastung der Flüssigphase $< 0,48$ kg/m$^2$s erstaunlicherweise keinen erkennbaren Einfluß auf den Fettsäureumsatz, während im Bereich von $> 0,48$ kg/m$^2$s, wie zu erwarten, der Umsatz mit zunehmender Querschnittsbelastung sinkt.

Selbstverständlich sind auch alle anderen Reaktionen, die die genannten Voraussetzungen erfüllen, vorteilhaft nach dem beschriebenen neuartigen Verfahren durchführbar. Anwendungen liegen im Bereich höher siedender temperaturempfindlicher Edukte oder Produkte, wie z.B. im Arbeitsfeld der Zwischenprodukterzeugung für Waschmittel, Pharmaka und Kosmetika. Die angeführten Reaktionen sind nur Beispiele für eine große Zahl von Synthesen, die durch das hier vorgestellte neuartige Verfahren durchgeführt werden können.

**Versuchsbeispiele**

Zum einen wurden Versuche in einem satzweise betriebenen Reaktor zur Ermittlung der Kinetik der Fettsäureveresterung bei Strippbedingungen durchgeführt, zum anderen erfolgten Versuche in einem Laborversuchsreaktivstripper.

**Versuche im Satzreaktor**

Bei diesen Experimenten wurde in einem Satzreaktor Fettsäure und Fettalkohol vorgelegt und in Anwesenheit eines geeigneten Katalysators die Veresterungsreaktion durchgeführt. Der saure Katalysator (Ionentauscher) wurde in Form von Raschig-Ringen als Festbett in den Reaktor eingebracht. Dabei wurde in einem Reaktionsvolumen von ca. 400 ml ca. 85 ml fester Katalysator eingebracht. Das Volumen der Flüssigphase betrug ca. 230 ml. Der Rest ist das Volumen des Strippgases. Als Strippgas wurde Stickstoff verwendet. Der Satzreaktor simuliert einen Abschnitt in einem

kolonnenförmigen Reaktivstripper.

Die hier gewählten Volumenverhältnisse von Katalysator: Flüssigphase : Gasphase sind auf einen als Kolonne betriebenen Reaktivstripper nicht übertragbar. Trotz der ungünstigen Einstellung hinsichtlich der relativ geringen Katalysatorkonzentration in bezug auf die Flüssigphase zeigten diese Versuche, daß das erfindungsgemäße Verfahren vorteilhaft arbeitet. Bei diesen Experimenten wurde die Reaktionstemperatur, das Einsatzverhältnis der Reaktanden sowie der Strippgasvolumenstrom variiert.

| Reaktionszeit [min] | Fettsäureumsatz [-] bei äquimolaren Einsatzverhältnis | |
|---|---|---|
| | 0; 17; 60 l $N_2$/h | 120 l $N_2$/h (Strippgasvolumenstrom) |
| 20 min | ≈0,2 | 0,5 |
| 40 min | ≈0,4 | 0,7 |
| 75 min | ≈0,62 | 0,8 |

Es wurde deutlich, daß der Umsatz an Ester in Abhängigkeit von der Reaktionszeit erheblich steigt, wenn gleichzeitig Strippgas durch den Satzreaktor geleitet wird. Dies ist auf die erfindungsgemäß verbesserte Entfernung des Koppelproduktes Wasser durch den Strippgasstrom zurückzuführen. Mit steigendem Strippgasstrom verbessert sich der Umsatz über der Zeit. Bei sehr geringen Strippgasstömen tritt die Wirkung des gleichzeitigen Strippens in einem Satzreaktor noch nicht in Erscheinung (kleiner 60 l/h Stickstoff). Aus diesen Experimenten im Satzreaktor ergibt sich, daß der Strippgasstrom (in diesem Falle Stickstoff) bei 110 °C Reaktionstemperatur und atmosphärischem Druck mehr als ca. 0,025 kg Stickstoff/ ($m^2$s) betragen sollte. Bezugsfläche ist die Querschnittsfläche des leeren Reaktivstrippers. Diese Experimente wurden so ausgeführt, daß das zu entfernende Nebenprodukt Wasser über seiner Siedetemperatur anfällt. Dies stellt den verfahrenstechnisch günstigeren Fall des "Verdampfungsstrippens" dar.

In einer zweiten Versuchsserie wurde 80 °C als Reaktionstemperatur bei atmosphärischem Druck eingestellt. Hierbei fiel Wasser unterhalb seiner Siedetemperatur flüssig an. Auch hier zeigte sich der Effekt, daß durch gleichzeitiges Strippen der Umsatz gesteigert werden konnte. Die Strippwirkung war jedoch geringer.

| Reaktionszeit [min] | Fettsäureumsatz [-] bei äquimolaren Einsatzverhältnis 129 l $N_2$/h (Strippgasvolumenstrom) |
|---|---|
| 20 min | 0,07 |
| 40 min | 0,13 |
| 75 min | 0,25 |

**Versuche im Laborreaktivstripper:**

Zum Nachweis der Funktion des erfindungsgemäßen Strippverfahrens in einer Kolonne wurde ein Laborreaktivstripper entwickelt. In dieser Kolonne mit einem Innendurchmesser von 80 mm wurde bei den ersten Versuchen ein Katalysatorfestbett mit einer Länge von ca. 1 m aus sauren Ionentauschern in Form von Raschig-Ringen als Katalysator verwendet. Das Volumen der katalytischen Raschig-Ring Packung betrug ca. 5 l. Am Kopf des Apparates wurde Fettsäure und Fettalkohol äquimolar zudosiert. Von unten - im Gegenstrom - erfolgte die Einleitung des Strippgases. Bei den ersten Versuchen wurde Stickstoff als Strippgas verwendet.

| | | |
|---|---|---|
| | Querschnittsbelastung mit Flüssigphase (Fettsäure + Alkohol) | 0,24 kg/($m^2$s) |

(fortgesetzt)

| | | |
|---|---|---|
| 1. | Querschnittsbelastung mit Strippgas Stickstoff | 0,049 kg/($m^2$s) |
| 2. | Querschnittsbelastung mit Strippgas Stickstoff | 0,085 kg/($m^2$s) |
| 3. | Querschnittsbelastung mit Strippgas | 0 kg/($m^2$s) |
| | Reaktionstemperatur | 110°C |
| | Reaktionsdruck | atmospärisch |

Mit steigendem Strippgasstrom (zumindest für kleine bis mittlere Volumenströme) stieg der Umsatz der Fettsäure.

| Abhängigkeit des Umsatzes [-] vom Strippgasvolumenstrom nach 90 min Reaktionszeit bei $V_{l,ges}$ ≈5,1 l/h, T=110°C und atmosphärischem Druck | | | |
|---|---|---|---|
| 0 | Nl $N_2$/h | (3) | 0,15 |
| 1500-2000 | Nl $N_2$/h | (2) | 0,27 |

Weiterhin wurde festgestellt daß mit steigendem Strippgasstrom (zumindest für kleine bis mittlere Volumenströme) der Wassergehalt der Produkte abnimmt, die Qualität also steigt.

| Wassergehalt nach 85 min Reaktionszeit bei $V_{l,ges}$ ≈5,1 l/h, T=110°C und atmosphärischem Druck | | |
|---|---|---|
| 0,25 Gew% $H_2O$ | bei 1500-2000 Nl $N_2$/h | (2) |
| 0,39 Gew% $H_2O$ | bei 0 Nl $N_2$/h | (3) |

Weitere Experimente mit Wasserstoff als Strippgas erfolgten im Anschluß. Das Anfahrverhalten des Laborreaktivstrippers für zwei Druckniveaus wurde untersucht. Die Drücke wurden so gewählt, daß beim niedrigen Druck Wasser als Gas anfällt, während beim hohen Druck Wasser als Flüssigkeit gebildet wird. Die weiteren Versuchsparameter waren:

| | | |
|---|---|---|
| | Querschnittsbelastung mit Flüssigphase (= Fettsäure + Alkohol) | 0,48 - 0,54 kg/($m^2$s) |
| 1. | Querschnittsbelastung mit Strippgas $H_2$ bei 2 bar | $3,15 \cdot 10^{-3}$ kg/($m^2$s) |
| 2. | Querschnittsbelastung mit Strippgas $H_2$ bei 0,35 bar | $1,42 \cdot 10^{-3}$ kg/($m^2$s) |
| | Reaktionstemperatur | 110°C |
| 1. | Reaktionsdruck | 2 bar Überdruck |
| 2. | Reaktionsdruck | 0,35 bar Überdruck |

Auch hier zeigt sich wie schon in den Versuchen im Satzreaktor, daß die Wirksamkeit des Strippens bei der Verfahrensführung "Verdampfungsstrippen" höher ist, als bei der Prozeßführung, bei der Wasser als Flüssigkeit anfällt.

| Abhängigkeit des Umsatzes [-] vom Strippgasvolumenstrom und Druckniveau nach 90 min Reaktionszeit bei $V_{l,ges}$ ≈10-11 l/h und T=110°C | | |
|---|---|---|
| 300 Nl $H_2$/h bei $p_Ü$≈2 bar | (1) | 0,082 |
| 300 Nl $H_2$/h bei $p_Ü$≈0,35 bar | (2) | 0,118 |

Weiterhin ist die Abhängigkeit der Verweilzeit der Flüssigphase sowie der Flüssigkeits hold-up in Abhängigkeit vom Flüssigplasevolumenstrom untersucht worden. Bei kleinen Belastungen des Reaktivstrippers mit Flüssigkeit sank der „hold-up" und die Verweilzeit stieg. Bei niedrigen Flüssigkeitsbelastungen konnte durch den Strippgasvolumenstrom eine deutliche Beeinflussung der Verweilzeit der Flüssigphase erreicht werden. Bei einer starken Variation der Flüssigkeitsbelastung (hier ca. 1 : 300) war immer noch ein einwandfreier Betrieb des Reaktivstrippers möglich. Auch der Strippgasvolumenstrom konnte in einem sehr weiten Bereich von 0 bis ca. 1000 l/h variiert werden, ohne daß der Prozeß aus hydrodynamischen Gründen zum Erliegen kommt.

Weitere Experimente zur Veresterung von Fettsäuren und Fettalkoholen unter Verwendung eines sauren Ionenaustauschers (Amberlist) mit Wasserstoff als Strippgas erfolgten in der Technikumsanlage. Es wurde mit einem Überschuß von 10% Fettalkohol gearbeitet. Um die maximale Umsatzrate zu bestimmen wurden mehrere Durchläufe bei konstanten Reaktionsparametern gefahren, wobei das jeweilige Veresterungsprodukt erneut eingesetzt wurde.

Tabelle 1

| Umsatz in Abhängigkeit von den Durchläufen | | | |
|---|---|---|---|
| Versuchs-Nr. | Umsetzung [%] | | |
| | (1) | (2) | (3) |
| 1 | 53,6 | 62,3 | 62,3 |
| 2 | 76 | 87 | 81,9 |
| 3 | 89,8 | 90,6 | 89,6 |
| 4 | 94,4 | 90,9 | 92,8 |
| 5 | 96,6 | | 93,3 |

Tabelle 2

| Parametereinstellungen Strippgasvol.-Strom 900 l$H_2$/h | | |
|---|---|---|
| Versuchs-Nr. | Druck | Temperatur |
| 1 | 0,35 bar ü | 140 °C |
| 2 | 0,5 bar ü | 160 °C |
| 3 | 3 bar ü | 180 °C |

**Patentansprüche**

1. Verfahren in einer Reaktionskolonne zur chemischen Umsetzung von Stoffen, deren Reaktion durch eine ungünstige Gleichgewichtslage der Bildungsreaktion oder ein vorgelagertes Gleichgewicht beschränkt ist, wobei während der Umsetzung durch einen oder mehrere Hilfsstoffe kontinuierlich ein oder mehrere abzutrennende Stoffe aus der Reaktionsmischung entfernt werden, **dadurch gekennzeichnet**, daß man durch die Reaktionskolonne, in

der ein Feststoff-Katalysator in einem Festbett angeordnet ist

- ein Strippgas, nämlich ein Permanentgas oder ein Gemisch von Permanentgasen als Hilfsstoff leitet, und
- Temperatur und Druck so einstellt, daß alle Edukte als Flüssigkeiten oder als Lösungen von Feststoffen und der oder die abzutrennenden Stoffe überwiegend gasförmig/dampfförmig in der Reaktionskolonne vorliegen.

**2.** Verfahren nach Auspruch 1, **dadurch gekennzeichnet**, daß man als Strippgas Wasserstoff einsetzt.

**3.** Verfahren nach einem der vorhergehenden Ansprüche, **dadurch gekennzeichnet**, daß man zur Entfernung des abzutrennenden Stoffes zusätzlich ein Schleppmittel einsetzt.

**4.** Verfahren nach Anspruch 3, **dadurch gekennzeichnet**, daß der abzutrennende Stoff zusätzlich durch Kohlenwasserstoffe wie Hexan, Benzol und/oder Toluol, besonders bevorzugt Hexan, als Schleppmittel entfernt wird.

**5.** Verfahren nach einem der vorhergehenden Ansprüche, **dadurch gekennzeichnet**, daß die durchzuführende Reaktion eine Kondensationsreaktion ist und das abzutrennende Kondensationsprodukt eine Molmasse von weniger als 100 g/mol hat.

**6.** Verfahren gemäß Anspruch 5, **dadurch gekennzeichnet**, daß man Carbonsäuren mit Alkoholen zu Carbonsäureestern verestert.

**7.** Verfahren gemäß Anspruch 5, **dadurch gekennzeichnet**, daß man Carbonsäureester mit Alkoholen zu Carbonsäurestern umestert.

**8.** Verfahren gemäß Anspruch 6, **dadurch gekennzeichnet**, daß man C6- bis C22- Carbonsäuren, insbesondere C8 bis C22, mit C1- bis C22- Alkoholen, insbesondere C8 bis C22, zu Carbonsäureestern verestert.

**9.** Verfahren gemäß Anspruch 7, **dadurch gekennzeichnet**, daß man Carbonsäureester mit einer C6- bis C22-Säuregruppe, insbesondere C8 bis C22, und einer C1- bis C4-Alkoholgruppe mit C6- bis C22-Alkoholen, insbesondere C8-C22, zu Carbonsäureestern umestert.

**10.** Verfahren nach einem der vorhergehenden Ansprüche, **dadurch gekennzeichnet**, daß ein saurer Feststoff-Katalysator in der Reaktionskolonne eingesetzt wird.

**11.** Verfahren nach einem der vorhergehenden Ansprüche, **dadurch gekennzeichnet**, daß unter Verwendung von Wasserstoff als Permanentgas mit Hilfe von an sich bekannten Katalysatoren in der Reaktionskolonne gleichzeitig die Hydrierung von Doppelbindungen erfolgt.

**12.** Verfahren nach Anspruch 6 bis 11, **dadurch gekennzeichnet**, daß man einen mehrfunktionellen Katalysator in der Reaktionskolonne einsetzt, der die Reaktionen Veresterung und Hydrierung und/oder Isomerisierung unterstützt.

**13.** Verfahren nach einem der vorhergehenden Ansprüche, **dadurch gekennzeichnet**, daß man das Strippgas, ggf. zusammen mit dem Schlepper, im Gegenstrom von unten nach oben durch die Reaktionskolonne führt, den abzutrennenden Stoff von dem Strippgas und ggf. dem Schlepper in einem Kondensator abtrennt und das Strippgas und ggf. den Schlepper oder einen Teil derselben in die Reaktionskolonne zurückführt.

## Claims

**1.** A process carried out in a reaction column for the chemical reaction of substances the reaction of which is affected by an unfavorable equilibrium position of the main reaction or a preceding equilibrium, wherein during the reaction one or more substances to be separated are continuously removed from the reaction mixture by one or more auxiliaries,
**characterized in that**
through the reaction column wherein a solid catalyst is arranged as a fixed bed

- a stripping gas which is a permanent gas or a mixture of permanent gases is led as an auxiliary and
- temperature and pressure are adjusted such that in the reaction column all the educts are present as liquids or solutions of solids and the substance(s) to be separated is (are) predominantly

gaseous/vaporous.

**2.** A process according to claim 1,
**characterized in that**
hydrogen is employed as a stripping gas.

**3.** A process according to any one of the preceding claims,
**characterized in that**
an entraining agent is additionally used in order to remove the substance to be separated.

**4.** A process according to claim 3,
**characterized in that**
the substance to be separated is additionally removed by employing hydrocarbons, such as hexane, benzene and/or toluene, hexane being particularly preferred, as entraining agents.

**5.** A process according to any one of the preceding claims,
**characterized in that**
the reaction to be carried out is a condensation reaction and the condensation product to be separated has a molecular mass of less than 100 g/ mole.

**6.** A process according to claim 5,
**characterized in that**
carboxylic acids are esterified with alcohols to form carboxylic acid esters.

**7.** A process according to claim 5,
**characterized in that**
carboxylic acid esters are transesterified with alcohols to form carboxylic acid esters.

**8.** A process according to claim 6,
**characterized in that**
$C_6$ - $C_{22}$ carboxylic acids, particularly $C_8$ - $C_{22}$ carboxylic acids, are esterified with $C_1$ to $C_{22}$ alcohols, particularly $C_8$ to $C_{22}$ alcohols, to form carboxylic acid esters.

**9.** A process according to claim 7,
**characterized in that**
carboxylic acid esters having a $C_6$ - $C_{22}$ acid group, particularly $C_8$ - $C_{22}$, and a $C_1$ to $C_4$ alcohol group are transesterified with $C_6$ to $C_{22}$ alcohols, particularly $C_8$ to $C_{22}$, to form carboxylic acid esters.

**10.** A process according to any one of the preceding claims,
**characterized in that**
an acidic solid catalyst is employed in the reaction column.

**11.** A process according to any one of the preceding claims,
**characterized in that**
double bonds are simultaneously hydrogenated in the reaction column by using hydrogen as a permanent gas and catalysts known in the art.

**12.** A process according to claims 6 to 11,
**characterized in that**
a multifunctional catalyst supporting the esterification and hydrogenation and/or isomerization reactions is employed in the reaction column.

**13.** A process according to any one of the preceding claims,
**characterized in that**
the stripping gas, optionally jointly with the entraining agent, is (are) countercurrently led through the reaction column from the bottom to the top, the substance to be separated is removed from the stripping gas and, optionally, the entraining agent in a condenser, and the stripping gas and, optionally, the entraining agent, or part thereof is (are) returned to the reaction column.

## Revendications

1. Procédé dans une colonne de réaction pour la transformation chimique de matières, dont la réaction est limitée par un état d'équilibre défavorable de la réaction de formation ou par un équilibre préétabli, dans lequel une ou plusieurs matières à séparer sont éliminées de manière continue du mélange réactionnel pendant la transformation au moyen d'un ou de plusieurs agents auxiliaires, caractérisé en ce que, par la colonne de réaction, dans laquelle un catalyseur solide est agencé dans un lit fixe :

   - on conduit un gaz de stripping, à savoir un gaz permanent ou un mélange de gaz permanents en tant qu'agent auxiliaire, et
   - on règle la température et la pression de sorte que tous les éduits sont présents dans la colonne de réaction sous forme de liquides ou sous forme de solutions de solides et la ou les matières à séparer sont principalement présentes sous forme gazeuse/vapeur.

2. Procédé suivant la revendication 1, caractérisé en ce qu'on utilise de l'hydrogène comme gaz de stripping.

3. Procédé suivant l'une des revendications précédentes, caractérisé en ce qu'on utilise en outre pour l'élimination de la matière à séparer un agent d'entraînement.

4. Procédé suivant la revendication 3, caractérisé en ce que la matière à séparer est en outre éliminée par des hydrocarbures comme l'hexane, le benzène et/ou le toluène, de manière particulièrement préférée l'hexane, en tant qu'agent d'entraînement.

5. Procédé suivant l'une des revendications précédentes, caractérisé en ce que la réaction à réaliser est une réaction de condensation et en ce que le produit de condensation à séparer a une masse molaire inférieure à 100 g/mole.

6. Procédé suivant la revendication 5, caractérisé en ce qu'on estérifie des acides carboxyliques avec des alcools en esters d'acide carboxylique.

7. Procédé suivant la revendication 5, caractérisé en ce qu'on transestérifie des esters d'acide carboxylique avec des alcools en esters d'acide carboxylique.

8. Procédé suivant la revendication 6, caractérisé en ce qu'on estérifie des acides carboxyliques en $C_6$ à $C_{22}$, en particulier en $C_8$ à $C_{22}$, avec des alcools en $C_1$ à $C_{22}$, en particulier en $C_8$ à $C_{22}$, en esters d'acide carboxylique.

9. Procédé suivant la revendication 7, caractérisé en ce qu'on transestérifie des esters d'acide carboxylique avec un groupement acide en $C_6$ à $C_{22}$, en particulier en $C_8$ à $C_{22}$ et un groupement alcool en $C_1$ à $C_4$ avec des alcools en $C_6$ à $C_{22}$, en particulier en $C_8$ à $C_{22}$, en esters d'acide carboxylique.

10. Procédé suivant l'une des revendications précédentes, caractérisé en ce qu'on utilise dans la colonne de réaction un catalyseur solide acide.

11. Procédé suivant l'une des revendications précédentes, caractérisé en ce que l'hydrogénation de doubles liaisons s'effectue simultanément dans la colonne de réaction en utilisant de l'hydrogène comme gaz permanent avec l'aide de catalyseurs connus en soi.

12. Procédé suivant les revendications 6 à 11, caractérisé en ce qu'on utilise dans la colonne de réaction un catalyseur multifonctionnel qui assiste les réactions d'estérification et d'hydrogénation et/ou d'isomérisation.

13. Procédé suivant l'une des revendications précédentes, caractérisé en ce qu'on conduit le gaz de stripping, le cas échéant conjointement avec l'agent d'entraînement, à contre-courant du bas vers le haut à travers la colonne de réaction, en ce qu'on sépare dans un condenseur la matière à séparer du gaz de stripping et, le cas échéant, de l'agent d'entraînement, en ce que le gaz de stripping et, le cas échéant, l'agent d'entraînement ou une partie de ces derniers sont ramenés dans la colonne de réaction.

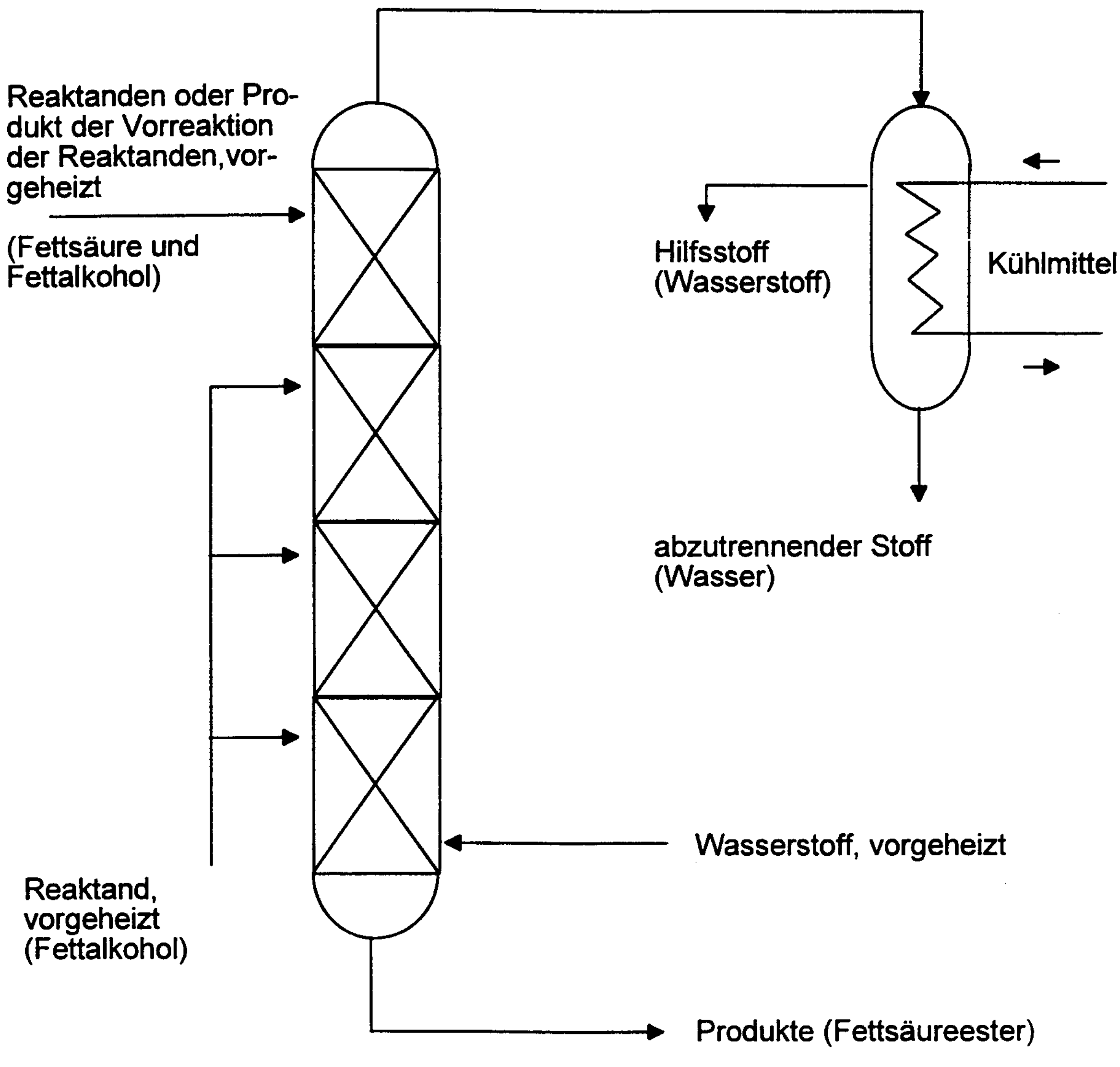


Abbildung 1: Prinzip des Reaktionsstrippers (am Beispiel der Herstellung von Fettsäureestern aus Fettsäuren und Fettalkoholen)